# EUROPEAN PATENT APPLICATION

(11) **EP 4 242 234 A1**
(43) Date of publication of application: **13.09.2023**
(21) Application number: 21902427.0
(22) Date of filing: 29.11.2021
(51) Int. Cl.: C07K 16/28, C12N 15/13, C12N 15/85, C12N 5/10, A61K 39/395, A61P 35/00

(54) **ANTI-LAG-3 MONOCLONAL ANTIBODY, AND ANTIGEN-BINDING FRAGMENT AND APPLICATION THEREOF**

(30) Priority: 10.12.2020 CN 202011436581
(71) Applicant: Beijing Dongfang Biotech Co. Ltd., Beijing 100176 (CN); Beijing Jingyitaixiang Technology Development Co. Ltd., Beijing 100176 (CN)
(72) Inventor: BAI, Yi, Beijing 100176 (CN); ZHOU, Junjie, Beijing 100176 (CN); LIU, Si, Beijing 100176 (CN)
(74) Representative: Delbarba, Andrea
(86) International application number: PCT/CN2021/133941
(87) International publication number: WO 2022/121720

(57) **Abstract**

The present application relates to the field of biomedicine, and particularly provides an anti-LAG-3 monoclonal antibody and an antigen binding fragment thereof, and use thereof, wherein the anti-LAG-3 monoclonal antibody and the antigen binding fragment thereof comprise a heavy chain variable region and a light chain variable region, and are selected from any one of A-I, A-2, A-3, and A-4. The provided monoclonal antibody may be specifically bound to LAG-3, has a relatively high affinity and a very good biological activity, and may be used for treating various cancers or immunological diseases. The cancer comprises leukemia, lung cancer, stomach cancer, esophageal cancer, ovarian cancer, head and neck cancer, melanoma, kidney cancer, breast cancer, colorectal cancer, liver cancer, pancreatic cancer or bladder cancer; and the immunological disease comprises psoriasis, Crohn's disease, rheumatoid arthritis, primary biliary cirrhosis, systemic lupus erythematosus, multiple sclerosis, ulcerative colitis, and autoimmune hepatitis.

## Description

### Cross-Reference to Related Application

The present application claims priority of Chinese patent application 202011436581.5 filed on December 10, 2020, which is incorporated by reference herein.

### Technical Field

The present invention relates to the technical filed of biological medicine, and particularly relates to an anti-LAG-3 monoclonal antibody and an antigen binding fragment thereof, and use thereof.

### Background

Immunotherapy is a most popular cancer treatment mode at present and known as a third revolution of cancer treatment. The so-called "cancer immunotherapy" is a therapeutic method for attacking cancer cells by means of an autoimmune system of human body. A balance between the immune system and the cancer cells is a dynamic process of long-term gambling, and is both positively engaged and interwoven. Immune cells of a healthy body may discover and kill the cancer cells, but under an induction of various innate and acquired factors, the immune system may lose an absolute advantage and is even "instigated" by the cancer cells, and helps the occurrence and development of cancer. A targeting drug has been hoped for its accuracy, but cancer cells are prone to develop drug resistance due to their fickle and complexity. Therefore, the accuracy is also easily useless. For this reason, immune checkpoint inhibitors in the cancer treatment have gradually become a research hotspot since CTLA-4 and PD-1 are discovered.

With the advent of an immunological anti-cancer therapy, lymphocyte-activation gene 3 (LAG-3) is gradually recognized as a potential immune checkpoint receptor. It is reported that the LAG-3 plays an important role in promoting an activity of regulatory T cells and down-regulating activation and proliferation of T cells (Workman CJ et al., J. Immunol., 2005; 174: 688-695). The LAG-3 is also known as CD223. The gene comprises 8 exons, and is located on human chromosome 12 (mouse chromosome 6). The LAG-3 belongs to the immunoglobulin superfamily, and consists of 3 parts of an extracellular region, a transmembrane region, and a cytoplasmic region. The gene encodes a type I transmembrane protein consisting of 498 amino acids. The LAG-3 is widely expressed on surfaces of activated NK cells, T cells and other immune cells, may negatively regulate lymphocyte functions, and has been proved by a part of studies to play an important role in the aspects of tumors and autoimmune diseases.

Preclinical studies show that inhibition of the LAG-3 enables T cells to regain cytotoxicity, thereby limiting growth of tumors and enhancing a killing effect of tumors. At the same time, the inhibition of the LAG-3 also reduces an ability of regulatory T cells in inhibiting an immune response. Therefore, the LAG-3 is an immune checkpoint receptor that may regulate functions of T cells, the inhibition of the LAG-3 is more beneficial to patients, particularly those with tumors containing LAG-3-expressing immune cells. Thus, the LAG-3 is considered a more attractive target than other immune checkpoint proteins. In the current second-generation immune checkpoint targets, the LAG-3 is a target with more clinical data and relatively determined druggability. In view of the above importance of the existence of the LAG-3, in order to meet needs of patients with tumors or other immunological diseases at home and abroad, it is urgently needed to develop an anti-LAG-3 monoclonal antibody with a relatively high biological activity.

### Summary

In order to meet needs of the domestic market, the present invention obtains an anti-LAG-3 monoclonal antibody or an antigen binding fragment thereof which may specifically bind to LAG-3 and has a relatively high biological activity through screening of an immune library.

Specific technical solutions of the present invention are as follows:
The present invention provides an anti-LAG-3 monoclonal antibody or an antigen binding fragment thereof, comprising a heavy chain variable region and a light chain variable region, wherein the heavy chain variable region comprises 3 heavy chain complementarity determining regions, respectively represented by HCDR1, HCDR2, and HCDR3, and the light chain variable region comprises 3 light chain complementarity determining regions, respectively represented by LCDR1, LCDR2, and LCDR3, and the monoclonal antibody or the antigen binding fragment thereof is selected from any one of the following:
A-1: the heavy chain complementarity determining region HCDR1 comprises an amino acid sequence shown in SEQ ID No: 1, the heavy chain complementarity determining region HCDR2 comprises an amino acid sequence shown in SEQ ID No: 2, the heavy chain complementarity determining region HCDR3 comprises an amino acid sequence shown in SEQ ID No: 3, the light chain complementarity determining region LCDR1 comprises an amino acid sequence shown in SEQ ID No: 4, the light chain complementarity determining region LCDR2 comprises an amino acid sequence shown in SEQ ID No: 5, and the light chain complementarity determining region LCDR3 comprises an amino acid sequence shown in SEQ ID No: 6;
A-2: the heavy chain complementarity determining region HCDR1 comprises an amino acid sequence shown in SEQ ID No: 1, the heavy chain complementarity determining region HCDR2 comprises an amino acid sequence shown in SEQ ID No: 2, the heavy chain complementarity determining region HCDR3 comprises an amino acid sequence shown in SEQ ID No: 3, the light chain complementarity determining region LCDR1 comprises an amino acid sequence shown in SEQ ID No: 4, the light chain complementarity determining region LCDR2 comprises an amino acid sequence shown in SEQ ID No: 7, and the light chain complementarity determining region LCDR3 comprises an amino acid sequence shown in SEQ ID No: 8;
A-3: the heavy chain complementarity determining region HCDR1 comprises an amino acid sequence shown in SEQ ID No: 9, the heavy chain complementarity determining region HCDR2 comprises an amino acid sequence shown in SEQ ID No: 10, the heavy chain complementarity determining region HCDR3 comprises an amino acid sequence shown in SEQ ID No: 11, the light chain complementarity determining region LCDR1 comprises an amino acid sequence shown in SEQ ID No: 12, the light chain complementarity determining region LCDR2 comprises an amino acid sequence shown in SEQ ID No: 13, and the light chain complementarity determining region LCDR3 comprises an amino acid sequence shown in SEQ ID No: 14; and
A-4: the heavy chain complementarity determining region HCDR1 comprises an amino acid sequence shown in SEQ ID No: 15, the heavy chain complementarity determining region HCDR2 comprises an amino acid sequence shown in SEQ ID No: 16, the heavy chain complementarity determining region HCDR3 comprises an amino acid sequence shown in SEQ ID No: 17, the light chain complementarity determining region LCDR1 comprises an amino acid sequence shown in SEQ ID No: 18, the light chain complementarity determining region LCDR2 comprises an amino acid sequence shown in SEQ ID No: 19, and the light chain complementarity determining region LCDR3 comprises an amino acid sequence shown in SEQ ID No: 20.

The anti-LAG-3 monoclonal antibody or the antigen binding fragment thereof provided by the present invention may specifically bind to LAG-3 and has a better binding activity. Blocking LAG-3 may reverse an inhibiting effect of LAG-3 on T cells, enhance the activity of the T cells, reduce the number of regulatory T cells, and also improve sensitivity of a T cell immune response, thereby may be used for treating immunological diseases or cancers.

Further, the monoclonal antibody or the antigen binding fragment thereof is a murine antibody molecule, and the murine antibody molecule is selected from any one of the following:
MA-1: the heavy chain variable region comprises an amino acid sequence shown in SEQ ID No: 21, and the light chain variable region comprises an amino acid sequence shown in SEQ ID No: 22;
MA-2: the heavy chain variable region comprises an amino acid sequence shown in SEQ ID No: 21, and the light chain variable region comprises an amino acid sequence shown in SEQ ID No: 23;
MA-3: the heavy chain variable region comprises an amino acid sequence shown in SEQ ID No: 24, and the light chain variable region comprises an amino acid sequence shown in SEQ ID No: 25; and
MA-4: the heavy chain variable region comprises an amino acid sequence shown in SEQ ID No: 26, and the light chain variable region comprises an amino acid sequence shown in SEQ ID No: 27; and
   preferably, the murine antibody molecule is the MA-1.

In the present invention, mice are immunized with an LAG-3 antigen through mouse immunization, an immunization method is optimized, a phage display library is established, and the murine antibody molecule with a relatively high affinity, better activity and stability is screened out. A large number of cell-level experiments proved that compared with other 3 murine antibody molecules, MA-1 has a higher biological activity. Therefore, the MA-1 is preferably selected in the present invention.

Further, the murine antibody molecule further comprises a heavy chain constant region and a light chain constant region, the heavy chain constant region is one of murine IgG1 type, !gG2a type, IgG2b type, and IgG3 type, the heavy chain constant region of the IgG1 type has an amino acid sequence shown in SEQ ID No: 29, the heavy chain constant region of the IgG2a type has an amino acid sequence shown in SEQ ID No: 30, the heavy chain constant region of the IgG2b type has an amino acid sequence shown in SEQ ID No: 31, and the heavy chain constant region of the IgG3 type has an amino acid sequence shown in SEQ ID No: 32; and the light chain constant region is a murine Cₖ chain with an amino acid sequence shown in SEQ ID No: 28; and
preferably, the heavy chain constant region is the murine IgG1 type.

Further, the monoclonal antibody or the antigen binding fragment thereof is a chimeric antibody molecule which comprises a heavy chain variable region of the murine antibody molecule, a light chain variable region of the murine antibody molecule, and a humanized antibody constant region; the humanized antibody constant region comprises a humanized antibody heavy chain constant region and a humanized antibody light chain constant region, the humanized antibody heavy chain constant region is one of human IgG1 type, IgG2 type, or IgG4 type, the heavy chain constant region of the IgG1 type has an amino acid sequence shown in SEQ ID No: 39, the heavy chain constant region of the IgG2 type has an amino acid sequence shown in SEQ ID No: 40, and the heavy chain constant region of the IgG4 type has an amino acid sequence shown in SEQ ID No: 41; and the humanized antibody light chain constant region is a human Cₖ chain having an amino acid sequence shown in SEQ ID No: 42; and
preferably, the humanized antibody heavy chain constant region is the human IgG4 type.

The chimeric antibody molecule comprises the variable region sequence of the murine antibody molecule and the constant region of the human antibody, and the design of the chimeric antibody molecule is used to verify that the constant region of the present invention does not change the function of the CDR after humanization, which provides the basis for further research and development on the humanized antibody molecule.

Further, the monoclonal antibody or the antigen binding fragment thereof is a humanized antibody molecule, and the humanized antibody molecule is selected from any one of the following:
HA-1: the heavy chain variable region comprises an amino acid sequence shown in SEQ ID No: 33, and the light chain variable region comprises an amino acid sequence shown in SEQ ID No: 34;
HA-2: the heavy chain variable region comprises an amino acid sequence shown in SEQ ID No: 33, and the light chain variable region comprises an amino acid sequence shown in SEQ ID No: 35;
HA-3: the heavy chain variable region comprises an amino acid sequence shown in SEQ ID No: 36, and the light chain variable region comprises an amino acid sequence shown in SEQ ID No: 35;
HA-4: the heavy chain variable region comprises an amino acid sequence shown in SEQ ID No: 36, and the light chain variable region comprises an amino acid sequence shown in SEQ ID No: 34; and
HA-5: the heavy chain variable region comprises an amino acid sequence shown in SEQ ID No: 37, and the light chain variable region comprises an amino acid sequence shown in SEQ ID No: 38; and
   preferably, the humanized antibody molecule is the HA-1.

According to the present invention, the humanized antibody molecule is obtained after humanization screening. In-vivo and in-vitro experimental verifications show that in the 5 humanized antibody molecules provided by the present invention, HA-1 has a relatively high activity and a most significant drug effect, such that HA-1 is preferably selected in the present invention.

Further, the humanized antibody molecule further comprises a heavy chain constant region and a light chain constant region, the heavy chain constant region is one of human IgG1 type, IgG2 type, or IgG4 type, the heavy chain constant region of the IgG1 type has an amino acid sequence shown in SEQ ID No: 39, the heavy chain constant region of the IgG2 type has an amino acid sequence shown in SEQ ID No: 40, the heavy chain constant region of the IgG4 type has an amino acid sequence shown in SEQ ID No: 41, and the light chain constant region is a human Cₖ chain with an amino acid sequence shown in SEQ ID No: 42; and
preferably, the heavy chain constant region is the human IgG4 type.

Further, the humanized antibody molecule is a full-length antibody or an antibody fragment, the humanized antibody molecule comprises one or a combination of more of Fab, F(ab)2, Fv or ScFv.

The present invention further provides a polypeptide or a protein, comprising the anti-LAG-3 monoclonal antibody or the antigen binding fragment thereof.

The present invention further provides a polynucleotide sequence or combination, encoding an amino acid sequence of the anti-LAG-3 monoclonal antibody or the antigen-binding.

The present invention further provides a recombinant DNA expression vector, comprising the polynucleotide sequence or combination.

The present invention further provides a host cell transfected with the recombinant DNA expression vector, wherein the host cell comprises a prokaryotic cell, a yeast cell, an insect cell, or a mammalian cell; and
preferably, the host cell is the mammalian cell, and the mammalian cell is an HEK293E cell, a CHO cell, or an NS0 cell.

The present invention further provides a drug or a pharmaceutical composition, comprising the anti-LAG-3 monoclonal antibody or the antigen binding fragment thereof.

The present invention further provides use of the anti-LAG-3 monoclonal antibody or the antigen binding fragment thereof in the preparation of a drug for treating cancers or immunological diseases;
preferably, the cancers comprises leukemia, lung cancer, stomach cancer, esophageal cancer, ovarian cancer, head and neck cancer, melanoma, kidney cancer, breast cancer, colorectal cancer, liver cancer, pancreatic cancer or bladder cancer; and
the immunological diseases comprises psoriasis, Crohn's disease, rheumatoid arthritis, primary biliary cirrhosis, systemic lupus erythematosus, multiple sclerosis, ulcerative colitis, and autoimmune hepatitis.

The present invention further provides use of the anti-LAG-3 monoclonal antibody or the antigen binding fragment thereof in combination with an anti-PD-1 monoclonal antibody in the preparation of a drug for treating a cancer or an immunological disease.

Further, the anti-PD-1 monoclonal antibody comprises DFPD1-9, DFPD1-10, DFPD1-11, DFPD1-12, DFPD1-13, nivolumab, pembrolizumab, toripalimab, sintilimab, tislelizumab, camrelizumab, penpulimab, or zimberelimab.

Preferably, the anti-PD-1 monoclonal antibody is DFPD1-10, and the DFPD1-10 comprises a light chain variable region shown in SEQ ID No: 45 and a heavy chain variable region shown in SEQ ID No: 43.

Preferably, the anti-PD-1 monoclonal antibody is nivolumab.

Preferably, the cancer is selected from leukemia, lung cancer, stomach cancer, esophageal cancer, ovarian cancer, head and neck cancer, melanoma, kidney cancer, breast cancer, colorectal cancer, liver cancer, pancreatic cancer or bladder cancer; and the immunological disease comprises psoriasis, Crohn's disease, rheumatoid arthritis, primary biliary cirrhosis, systemic lupus erythematosus, multiple sclerosis, ulcerative colitis, and autoimmune hepatitis.

The present invention has the following beneficial effects: the anti-LAG-3 monoclonal antibody or the antigen binding fragment thereof provided by the present invention may specifically bind to LAG-3, has a relatively high affinity and a very good biological activity, and may be used for treating various cancers or immunological diseases. The cancer includes but is not limited to leukemia, lung cancer, stomach cancer, esophageal cancer, ovarian cancer, head and neck cancer, melanoma, kidney cancer, breast cancer, colorectal cancer, liver cancer, pancreatic cancer or bladder cancer; and the immunological disease includes but is not limited to psoriasis, Crohn's disease, rheumatoid arthritis, primary biliary cirrhosis, systemic lupus erythematosus, multiple sclerosis, ulcerative colitis, and autoimmune hepatitis.

### Brief Description of the Drawings

Fig. 1 is a plasmid profile of the pScFv-Disb-HS vector in Example 2 of the present invention;
Fig. 2 shows comparison of relative affinities of anti-LAG-3 phage monoclonal antibodies inthe gradient-dilution ELISA in example 3 of the present invention;
Fig. 3 is a profile of a vector pTSE in Example 5 of the present invention;
Fig. 4 shows a denaturing polyacrylamide gel electrophoresis of murine antibody molecules in Example 5 of the present invention;
Fig. 5 shows comparison of binding abilities of murine antibodies to LAG-3 in Example 6 of the present invention;
Fig. 6 shows secretion of cytokine IL-2 by a murine antibody in a mixed lymphocyte reaction (MLR) in Example 7 of the present invention;
Fig. 7 shows a denaturing polyacrylamide gel electrophoresis of humanized antibody molecules in Example 12 of the present invention;
Fig. 8 shows comparison of binding abilities of humanized antibody molecules to LAG-3 in Example 15 of the present invention;
Fig. 9 shows activities of anti-LAG-3 humanized antibody molecules tested by an MLR in Example 16 of the present invention;
Fig. 10 shows a test effect of an anti-LAG-3 monoclonal antibody of Example 17 on inhibiting MC38 colorectal cancer in mice;
Fig. 11 shows an evaluation of molecular thermostability of an anti-LAG-3 monoclonal antibody, an HA-1 protein in Example 18 of the present invention;
Fig. 12 is a tumor volume growth curve graph of an anti-LAG-3 monoclonal antibody in combination with an anti-PD-1 monoclonal antibody in an MC38 colorectal cancer model in Example 22 of the present invention; and
Fig. 13 is a tumor weight column diagram of an anti-LAG-3 monoclonal antibody in combination with an anti-PD-1 monoclonal antibody in an MC38 colorectal cancer model in Example 22 of the present invention.

### Detailed Description of the Embodiments

In order to understand the present invention more easily, before the examples are described, some technical and scientific terms of the present invention are firstly illustrated below:
The term "LAG-3" used herein refers to lymphocyte-activation gene 3. The LAG-3 herein includes but is not limited to dimer-expressed LAG-3 (e.g., CD223 well-known in the art) on surfaces of activated T cells, NK cells, and B cells, and a soluble form of the LAG-3 found in human serum, all referred to herein as the LAG-3.

The term "antibody" used herein includes a whole antibody and any antigen binding fragment thereof. The antibody includes a murine antibody, a humanized antibody, a bispecific antibody or a chimeric antibody. Antibody may also be Fab, F(ab)2, Fv, or ScFv (single-chain antibody), and may be a naturally occurring antibody or also an altered (e.g., mutated, deleted, substituted, etc.) antibody.

The terms "variable region" and "constant region" used herein, i.e., a sequence region, near the N segments, of antibody heavy chain and light chain is a variable region (V region), and the rest amino acid sequence near the C segments is relatively stable, which is the constant region (C region). The variable region includes 3 complementarity determining regions (CDRs) and 4 framework regions (FRs), each light chain variable region and heavy chain variable region consists of 3 CDRs and 4 FRs, the 3 CDRs of a heavy chain are represented by HCDR1, HCDR2, and HCDR3, respectively, and the 3 CDRs of a light chain are represented by LCDR1, LCDR2, and LCDR3, respectively.

The term "murine antibody molecule" used herein is derived from an antibody obtained after immunization injection of a mouse with an LAG-3 antigen.

The term "chimeric antibody molecule" used herein refers to an antibody obtained by fusing the variable region of a murine antibody with the constant region of a humanized antibody, which may reduce the immune response induced by the murine antibody in a human body. The chimeric antibody is prepared by inserting light and heavy chain variable region genes of a murine monoclonal antibody into expression vectors containing humanized antibody constant regions, so that the variable regions of the light chain and the heavy chain in the expressed antibody molecule are murine, while the constant region is humanized, and near 2/3 part of the whole antibody molecule is humanized. The antibodies thus generated reduce immunogenicity of the murine antibody and at the same time retains the ability of the parental antibody to specifically bind to the antigen.

The term "humanized antibody molecule" used herein refers to grafting the CDRs of the murine monoclonal antibody to the variable regions of the humanized antibody to replace the CDRs of the humanized antibody, such that the humanized antibody may acquire an antigen binding specificity of the murine monoclonal antibody while reducing its heterogeneity.

The term "CHO cell" is a Chinese hamster ovary cell; the term "HEK 293E cell" refers to the human embryonic kidney 293E cell; and the term "NS0 cell" is a mouse NS0 thymoma cell.

The present invention is described in further detail below with reference to the examples.

### Example 1

Example 1 of the present invention provided an anti-LAG-3 monoclonal antibody or an antigen binding fragment thereof, specifically comprising a heavy chain variable region and a light chain variable region, wherein the heavy chain variable region comprised 3 heavy chain complementarity determining regions, respectively represented by HCDR1, HCDR2, and HCDR3, and the light chain variable region comprised 3 light chain complementarity determining regions, respectively represented by LCDR1, LCDR2, and LCDR3, and the monoclonal antibody or the antigen binding fragment thereof was selected from any one of the following:

| Combination | HCDR1 | HCDR2 | HCDR3 | LCDR1 | LCDR2 | LCDR3 |
|---|---|---|---|---|---|---|
| A-1 | DTYMH | KIDPANGNTKYDPKFQG | DTTVGLDY | RASSSVNYMY | YTSKLAS | QQWSSNPHT |
| | (SEQ ID No:1) | **(SEQ** ID No:2) | (SEQ ID No:3) | (SEQ ID No:4) | I (SEQ ID.No:5) | **(SEQ** ID No:6) |
| A-2 | DTYMH | KIDPANGNTKYDPKFQG | DTTVGLDY | RASSSVNYMY | YTSNLAP | QQFTSSPSMT |
| | (SEQ ID No:1) | (SEQ ID No:2) | (SEQ ID No:3) | (SEQ ID No:4) | SEO ID No:7 | (SEQ ID No:8) |
| A-3 | GYSFTSY | WIFPGTGNTRYNEKFKG | IGGRLTGDAMDY | RASESVDSYGNSFMH | LASNLES | QQNNEDPWT |
| | (SEQ ID No:9) | (SEQ ID No:10) | (SEQ ID No:11) | (SEQ ID No:12) | (SEQ ID No: 13 | **(SEQ** ID No: 14) |
| A-4 | TYAVH | VTWSGGSTDYNTAFISR | | TASSSVSSSYLH | STSNLAS | HQYHRSPPT |
| | (SEQ ID No**:**15) | (SEQ ID No 16) | (SEQ ID No: 17) | (SEQ ID No:18) | (SEQ ID No:19) | (SEQ ID No:20) |

### Example 2 Screening of murine antibody molecule

According to the present invention, mice were immunized with an LAG-3 antigen, an immunization method was optimized, a phage display library was established, and construction, screening, and identification of the specific phage display library were as follows:
Step 1: immunization of mice with LAG-3 antigen
1. Experimental animals:
   species and strains: female BALB/c mice;
   weight: 18-20 g; and
   Experimental animal provider: Beijing HFK Bioscience Co., Ltd.
2. Immunization: the mice were immunized,and the immune antigen was human LAG-3 (the gene was synthesized by GenScript Biotech Corporation (Nanjing), a vector was constructed, and the protein was expressed and purified by our company).

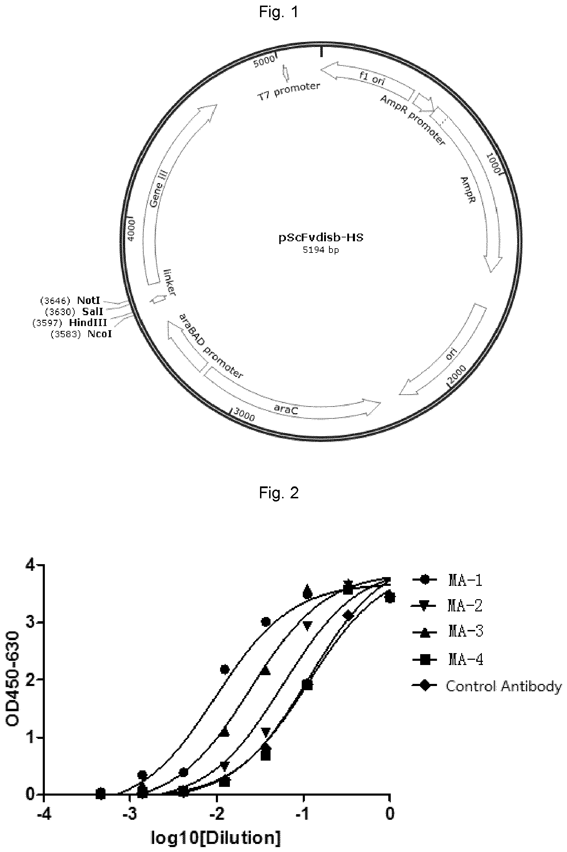

Step 2: construction of aphage antibody library
Mouse splenocytes with a higher titer was taken, total RNA in the mouse splenocytes was extracted with a Trizol reagent (purchased from Ambion with the Cat. No. 15596026), cDNA was obtained by RT-PCR, a PCR amplification was performed with cDNA as a template and degenerate primers (the used degenerate primers refer to a reference: Journal of Immunological Methods, 233(2000), 167-177), thus a heavy chain variable region gene library (VH) and a light chain variable region gene library (VL) of the immunized mouse antibody were obtained, a light chain and a heavy chain were double-enzyme-digested respectively, and connected to a vector which was also enzyme digested step-by-step to construct a pScFv-Disb-HS-VH-VL gene library, a PscFv-DisB-HS vector was modified from a pComb3 vector (purchased from the Biovector Science Lab, Inc) with a series of gene cloning methods, such that the vector was used for constructing and expressing a phage single-chain antibody library. The modified vector was named pScFv-Disb-HS vector, its obtained plasmid profile was shown in Fig. 1, and a mouse immune phage antibody library was constructed on the basis of the vector.
Step 3: an immune tube was coated with LAG-3 as an antigen,an amount of the antigen coatingis 5µg/500µL/tube, and coating was performed at 4°C overnight, the immune tube and the immune phage antibody library were blocked at room temperature for 1h with 4% skim milk powder/PBST respectively. The blocked immune phage antibody library was added into the immune tube with an input amount of phages of about 10⁹-10¹²for an antigen-antibody binding, after reacting at room temperature for 1h, unbound phages were washed away with PBST-PBS, eluting was performed with 0.1M of glycine-HCl at a pH of 2.2, and finally an eluted phage antibody solution was neutralized to a pH of 7.0 with 1.5M of tris-HCl at a pH of 8.8.
Step IV: 10 ml of a TG1 bacterial solution growing to a logarithmic phase was infected with the neutralized phages, let the solution stood in an incubator at 37°Cfor 30 min, a part of the bacterial solution was taken out for gradient dilution, and coated on a 2YTAG plate for the calculation of the yield of the phages. The remaining bacterial solution was centrifuged, a supernatant was discarded, and the bacterial precipitate was resuspended in a small amount of a culture medium, aspirated, and coated on a 2YTAG large plate in preparation for a next round of screening.
Step V: the infected plated bacteria were scraped from the large plate, inoculated to a 2YTAG liquid culture medium, shaken to a logarithmic phase, an M13KO7 helper phage was added for superinfection, the bacteria were cultured overnight at 220 rpm at 28°C to prepare the phages, and the phages were precipitated and purified by PEG/NaCl for a next round of screening to perform a round of enrichment screening of the phage library.
Step VI: screening of LAG-3 phage single-chain antibody positive clone: after a round of screening, a well-separated monoclonal colonies was picked, inoculated into a 96-deep-well plate containing a 2YTAG liquid culture medium, cultured at 37°Cunderthe condition of 220rpm to a logarithmic growth phase, about 10¹⁰ of the helper phages M13KO7 were added per well, and static infection was performed at 37°C for 30min. Centrifugation was performed at 4,000rpm for 15min, a supernatant was discarded, the bacterial precipitate was resuspended with 2YTAK, and the bacteria were cultured overnight.at 28°Cunder the condition of 220rpm. After centrifugation at 4,000rpm and 4°C for 15min, an amplified phage supernatant was sucked for an ELISA identification, finally four anti-LAG-3 murine antibody molecules with a relatively high affinity were screened, named as MA-1, MA-2, MA-3, and MA-4 respectively, the obtained monoclonal antibodies were subjected to gene sequencing to be determined as correct antibody sequences, and after sequencing, the sequences of the 4 monoclonal antibodies screened above were as follows:

| Murine antibody molecule | Heavy chain variable region sequence | Light chain variable region sequence |
|---|---|---|
| MA-1 | SEQ ID No:21 | SEQ ID No:22 |
| MA-2 | SEQ ID No:21 | SEQ ID No:23 |
| MA-3 | SEQ ID No:24 | SEQ ID No:25 |
| MA-4 | SEQ ID No:26 | SEQ ID No:27 |

Specifically, SEQ ID No:21 (amino acid sequence of heavy chain variable region of MA-1 and MA-2): 
EVQLQQSGAELVKPGASVKLSCTASGFNIKDTYMHWVKQRPEQGLEWIGKIDPANGNTKYDP
KFQGKATITADTSSNTAYLQLSRLTSEDTAVYYCARDTTVGLDYWGQGTTLTVSS;
SEQ ID No: 22 (amino acid sequence of light chain variable region of MA-1): 
DIVITQSTAIMSASLGEKVTMSCRASSSVNYMYWYQQKSDASPKLWIYYTSKLASGVPARFSG
SGSGTSYSLTISSMEAEDAATYYCQQWSSNPHTFGGGTKLEIK;
SEQ ID No: 23 (amino acid sequence of light chain variable region of MA-2): 
DIVLTQTPAIMSASLGEKVTMSCRASSSVNYMYWYQQKSDASPKLWIYYTSNLAPGVPARFS
GSGSGNSYSLTISSMEGEDAATYYCQQFTSSPSMTFGGGTKLEIK;
SEQ ID No: 24 (amino acid sequence of heavy chain variable region of MA-3): 
EVKLEESGPELVKPGASVKISCKASGYSFTSYYIHWVKQRPGQGLEWIGWIFPGTGNTRYNE
KFKGKATLTADTSSSTVYMQLSGLTSEDSAVYFCARIGGRLTGDAMDYWGQGTSVTVSS;
SEQ ID No: 25 (amino acid sequence of light chain variable region of MA-3): 
DIVLTQSTASLAVSLGQRATISCRASESVDSYGNSFMHWYQQKPGQPPKLLIYLASNLESGVP
ARFSGSGSRTDFTLTIDPVEADDAATYYCQQNNEDPWTFGGGTKLEIK;
SEQ ID No: 26 (amino acid sequence of heavy chain variable region of MA-4): 
QVKLEESGPGLVQPSQSLSITCTVSAFSLTTYAVHWVRQSPGKGLEWLGVIWSGGSTDYNTA
FISRLNITKDNSKSQVFFKMNSLQANDTAIYYCARLDGTFFDYWGQGTTLTVSS; and
SEQ ID No: 27 (amino acid sequence of light chain variable region of MA-4):

DIVLTQTPAIMSASLGERVTMTCTASSSVSSSYLHWYQQKPGSSPKLWIYSTSNLASGVPARF
SGSGSGTSYSLTISSMEAEDAATYYCHQYHRSPPTFGGGTKLEIK.

### Example 3 Comparison of affinities of anti-LAG-3 phage monoclonal antibodies by gradient-dilution

The 4 murine antibody molecules (MA-1, MA-2, MA-3, and MA-4) obtained in example 2 were subjected to display and purification of monoclonal phages, then the phages were subjected to a gradient-dilution ELISA experiment to identify an affinity, and an anti-LAG-3 monoclonal antibody provided in patent application CN105992595A was selected as a control antibody. The specific method was as follows:
100ng/well/100µl of LAG-3 was coated with a carbonate buffer solution at a pH of 9.6 overnight at 4°C and washed three times with PBST, the 4 phage monoclonal antibodies screened in example 2 were respectively diluted with PBST in a three-fold gradient, and 100µl of the diluted sample was added to each well and stood at room temperature for 1 hour. An ELISA plate was washed with PBST, and an HRP-anti-M13 monoclonal antibody diluted with the PBST was added to the ELISA plate and stood at room temperature for 1h. Color development was performed with a TMB color developing kit at room temperature for 10 minutes, and terminated with 2M of H₂SO₄. Reading was performed at 450nm/630nm, a corresponding EC50 value was calculated, and specific data was as follows:

| Clone | MA-1 | MA-2 | MA-3 | MA-4 | Control antibody |
|---|---|---|---|---|---|
| EC50 | 0.010 | 0.058 | 0.024 | 0.117 | 0.115 |

Based on the above data and Fig. 2, the 4 different murine antibody molecules screened in example 2 may all bind to LAG-3. The monoclonal antibodies provided by the present invention had a relatively high affinity to the LAG-3.

### Example 4

On the basis of example 2, example 4 of the present invention further defined that the murine antibody molecule further comprised a heavy chain constant region and a light chain constant region, wherein the heavy chain constant region was one of murine IgG1 type, !gG2a type, IgG2b type, and IgG3 type, the heavy chain constant region of the IgG1 type had an amino acid sequence shown in SEQ ID No: 29, the heavy chain constant region of the IgG2a type had an amino acid sequence shown in SEQ ID No: 30, the heavy chain constant region of the IgG2b type had an amino acid sequence shown in SEQ ID No: 31, and the heavy chain constant region of the IgG3 type had an amino acid sequence shown in SEQ ID No: 32; and the light chain constant region was a murine Cₖ chain with an amino acid sequence shown in SEQ ID No: 28. The specific sequences were as follows:
SEQ ID No: 28 (light chain constant region sequence of murine Cₖ chain): 
ADAAPTVSIFPPSSEQLTSGGASVVCFLNNFYPKDINVKWKIDGSERQNGVLNSWTDQDSKD
STYSMSSTLTLTKDEYERHNSYTCEATHKTSTSPIVKSFNRNEC;
SEQ ID No: 29 (heavy chain constant region sequence of murine IgGI type): 
AKTTPPSVYPLAPGSAAQTNSMVTLGCLVKGYFPEPVTVTWNSGSLSSGVHTFPAVLQSDLY
TLSSSVTVPSSTWPSETVTCNVAHPASSTKVDKKIVPRDCGCKPCICTVPEVSSVFIFPPKPKDVLTI
TLTPKVTCVVVDISKDDPEVQFSWFVDDVEVHTAQTQPREEQFNSTFRSVSELPIMHQDWLNGKE
FKCRVNSAAFPAPIEKTISKTKGRPKAPQVYTIPPPKEQMAKDKVSLTCMITDFFPEDITVEWQWN
GQPAENYKNTQPIMDTDGSYFVYSKLNVQKSNWEAGNTFTCSVLHEGLHNHHTEKSLSHSPG;
SEQ ID No: 30 (heavy chain constant region sequence of murine !gG2a type): 
AKTTAPSVYPLAPVCGDTTGSSVTLGCLVKGYFPEPVTLTWNSGSLSSGVHTFPAVLQSDLYT
LSSSVTVTSSTWPSQSITCNVAHPASSTKVDKKIEPRGPTIKPCPPCKCPAPNLLGGPSVFIFPPKIK
DVLMISLSPIVTCVVVDVSEDDPDVQISWFVNNVEVHTAQTQTHREDYNSTLRVVSALPIQHQDW
MSGKEFKCKVNNKDLPAPIERTISKPKGSVRAPQVYVLPPPEEEMTKKQVTLTCMVTDFMPEDIYV
EWTNNGKTELNYKNTEPVLDSDGSYFMYSKLRVEKKNWVERNSYSCSVVHEGLHNHHTTKSFSR
TPGK;
SEQ ID No: 31 (heavy chain constant region sequence of murine IgG2b type): 
AKTTPPSVYPLAPGCGDTTGSSVTLGCLVKGYFPESVTVTWNSGSLSSSVHTFPALLQSGLY
TMSSSVTVPSSTWPSQTVTCSVAHPASSTTVDKKLEPSGPISTINPCPPCKECHKCPAPNLEGGPS
VFIFPPNIKDVLMISLTPKVTCVVVDVSEDDPDVQISWFVNNVEVHTAQTQTHREDYNSTIRVVSTL
 
PIQHQDWMSGKEFKCKVNNKDLPSPIERTISKIKGLVRAPQVYILPPPAEQLSRKDVSLTCLVVGFN
PGDISVEWTSNGHTEENYKDTAPVLDSDGSYFIYSKLNMKTSKWEKTDSFSCNVRHEGLKNYYLK
KTISRSPGK; and
SEQ ID No: 32 (heavy chain constant region sequence of murine IgG3 type):

ATTTAPSVYPLVPGCSDTSGSSVTLGCLVKGYFPEPVTVKWNYGALSSGVRTVSSVLQSGFY
SLSSLVTVPSSTWPSQTVICNVAHPASKTELIKRIEPRIPKPSTPPGSSCPPGNILGGPSVFIFPPKP
KDALMISLTPKVTCVVVDVSEDDPDVHVSWFVDNKEVHTAWTQPREAQYNSTFRVVSALPIQHQD
WMRGKEFKCKVNNKALPAPIERTISKPKGRAQTPQVYTIPPPREQMSKKKVSLTCLVTNFFSEAISV
EWERNGELEQDYKNTPPILDSDGTYFLYSKLTVDTDSWLQGEIFTCSVVHEALHNHHTQKNLSRS
PELELNETCAEAQDGELDGLWTTITIFISLFLLSVCYSASVTLFKVKWIFSSVVQVKQTAIPDYRNMI
GQGA.

### Example 5 Preparation of anti-LAG-3 murine antibody molecule

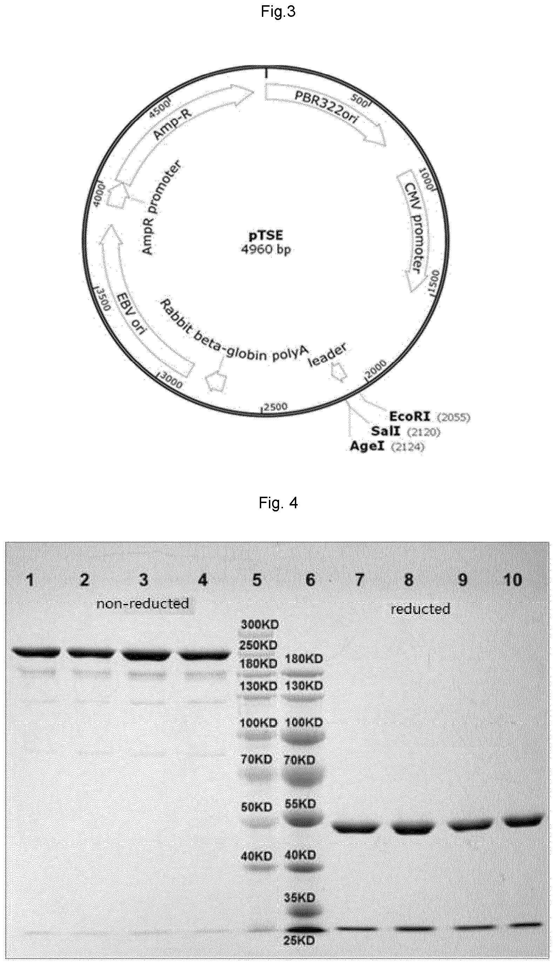

On the basis of example 4, example 5 of the present invention preferably defined that the heavy chain constant region of the murine antibody molecule was a murine IgG1 type, and the IgG1 type comprised the amino acid sequence shown in SEQ ID No: 29; and the light chain constant region was a murine Cₖ chain, and the murine Cₖ chain comprised an amino acid sequence shown in SEQ ID No: 28. A method for preparing the antibody specifically comprised the following steps:
1. Encoding genes of heavy chain VH and light chain VL of the 4 monoclonal antibodies screened in example 2 were respectively cloned into a pTSE vector (see Fig. 3) containing heavy chain and light chain constant region genes, preferably, a heavy chain constant region was the murine IgG1 type constant region (amino acid sequence shown in SEQ ID No: 29), a light chain constant region was the murine Cₖ chain (amino acid sequence shown in SEQ ID No: 28), and the structure of the pTSE vector was shown in Fig. 3 (a preparation process of the pTSE vector referred to page 3, paragraph [0019] of the description of CN103525868A).
2. The vector was transiently transfected into HEK293E cells (purchased from the Institute of Basic Medicine of Chinese Academy of Medical Sciences, Cat. No: GNHu43) for expressing an antibody, 4 monoclonal antibodies were obtained by a protein A affinity column purification using an AKTA instrument, a protein concentration was measured with a BCA kit (purchased from Beijing HuitianDongfang Technology Co., Ltd., Cat. No: BCA0020), and then a protein size was identified by SDS-PAGE. The results were shown in Fig. 4, non-reduced MA-1, MA-2, MA-3 and MA-4, protein molecular weight Marker1 and Marker2, and reduced MA-1, MA-2, MA-3 and MA-4 murine anti-LAG-3 monoclonal antibodies were sequentially present from left to right, and the molecular weight of each band was consistent with a theoretical value.

### Example 6 Binding experiment of murine antibody to LAG-3

100ng/well/100µl of LAG-3 was coated with a carbonate buffer solution at a pH of 9.6 overnight at 4°C. The LAG-3 was washed five times with 300µl/well of PBST, then 1% of BSA-PBST was added, the LAG-3 was blocked at 37°C for 1h, MA-1, MA-2, MA-3 and MA-4 murine antibodies with different dilution concentrations were added, wherein the 4 whole antibodies all had an initial highest concentration of 5µg/ml and respectively diluted by 3 times, and 12 gradients were made for each antibody, and incubated at 37°C for 1h. The obtained product was washed five times with 300µl/well of PBST, then Anti-Mouse Fc-HRP diluted with 1% BSA-PBST at 1:10,000 was added, and the mixture was incubated at 37°C for 1h. Color development was performed with 1 00pl/well of a TMB color developing kit at room temperature for 8 minutes, and terminated with 2M of H₂SO₄. Reading was performed at 450nm/630nm, a corresponding EC50 value was calculated, and specific data was as follows:

| Clone | MA-1 | MA-2 | MA-3 | MA-4 |
|---|---|---|---|---|
| EC50 (ng/ml) | 10.08 | 25.99 | 255.9 | 22.12 |

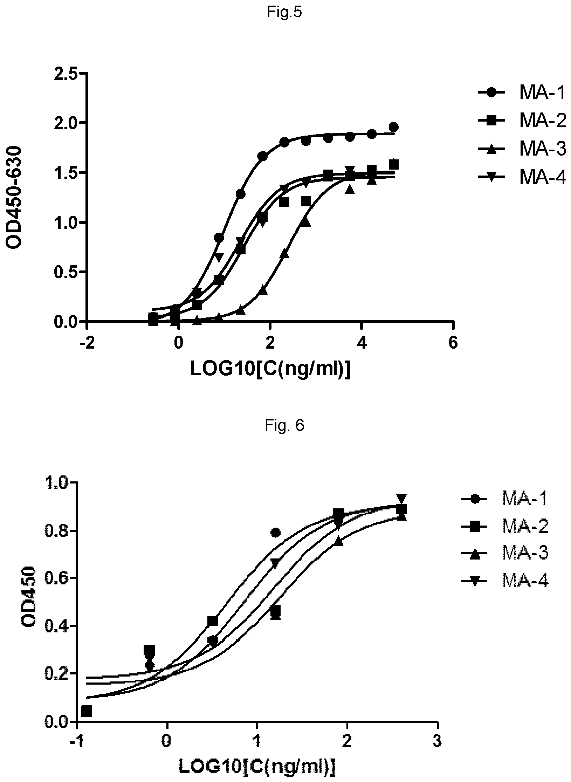

Based on the above data and Fig. 5, 4 different screened murine antibodies may all bind to LAG-3. In addition, the EC50 value of the MA-1 in the 4 murine antibody molecules was lowest. This indicated that the MA-1 had a best binding ability and highest affinity.

### Example 7 Effect of murine antibodies on secretion amount of cytokine IL-2 in mixed lymphocyte reaction

Fresh peripheral blood mononuclear cells (PBMCs) were separated by a density gradient centrifugation and CD14⁺ T cells were sorted by magnetic beads; and the CD14⁺ T cells were cultured by a culture medium of 20ng/mL of GM-CSF and 10ng/mL of IL-4, the culture medium was changed every 2 days, and the cells were induced into dendritic cells (DCs) in 7-10 days. Two days before the DCs were used, 25ng/mL of TNF-α was added to induce the DCs into mature DCs, and the mature DCs were collected and prepared into a cell suspension with a cell density of 1×10⁵ cells/mL.The CD⁴⁺ T cells were sorted from the PBMCs with magnetic beads, counted, and prepared into a cell suspension with a cell density of 1×10⁶ cells/mL. 100µl of each of the CD⁴⁺ T cells and the DCs were taken and added into a 96-well plate according to a ratio of 10:1.

The MA-1, MA-2, MA-3 and MA-4 murine antibodies prepared in example 5 were respectively subjected to a 4-fold gradient dilution, 6 gradients were set for each antibody, 50µl of each antibody was respectively taken and added into a 96-well plate, a concentration of IL-2 after culturing for 5 days was detected, reading was performed at 450nm, a corresponding EC50 value was calculated, and specific data was as follows:

| Clone | MA-1 | MA-2 | MA-3 | MA-4 |
|---|---|---|---|---|
| EC50 (µg/ml) | 0.024 | 0.030 | 0.076 | 0.121 |

Based on the data and Fig. 6, the 4 murine antibody molecules screened by the present invention all had a relatively good activity. In addition, it may further be obtained from the data that the EC50 value of the MA-1 was the lowest among the 4 monoclonal antibodies screened by the present invention, and thus the MA-1 had a best activity. Therefore, in the present invention the murine antibody molecule MA-1 may be humanized.

### Example 8

Example 8 of the present invention further defined that the monoclonal antibody or the antigen binding fragment thereof was a chimeric antibody molecule which comprised a heavy chain variable region of the murine antibody molecule, a light chain variable region of the murine antibody molecule, and a humanized antibody constant region in example 2; the humanized antibody constant region comprised a humanized antibody heavy chain constant region and a humanized antibody light chain constant region, the humanized antibody heavy chain constant region was one of human IgG1 type, IgG2 type, or IgG4 type, the heavy chain constant region of the IgG1 type had an amino acid sequence shown in SEQ ID No: 39, the heavy chain constant region of the IgG2 type had an amino acid sequence shown in SEQ ID No: 40, and the heavy chain constant region of the IgG4 type had an amino acid sequence shown in SEQ ID No: 41; and the humanized antibody light chain constant region was a human Cₖ chain having an amino acid sequence shown in SEQ ID No: 42.
SEQ ID No: 39 (amino acid sequence of heavy chain constant region of human IgGI type): 
ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSREEMTKNQVSLTCLVKGFYPSDI
AVEWESNGQPENNYKTTPPVLDSDGSFFLYSKLTVDKSRWQQGNVFSCSVMHEALHNHYTQKSL
SLSPGK;
SEQ ID No: 40 (amino acid sequence of heavy chain constant region of human IgG2 type): 
ASTKGPSVFPLAPCSRSTSESTAALGCLVKDYFPEPVTVSWNSGALTSGVHTFPAVLQSSGLY
SLSSVVTVPSSNFGTQTYTCNVDHKPSNTKVDKTVERKCCVECPPCPAPPVAGPSVFLFPPKPKD
 
TLMISRTPEVTCVVVDVSHEDPEVQFNWYVDGVEVHNAKTKPREEQFNSTFRVVSVLTVVHQDW
LNGKEYKCKVSNKGLPAPIEKTISKTKGQPREPQVYTLPPSREEMTKNQVSLTCLVKGFYPSDIAVE
WESNGQPENNYKTTPPMLDSDGSFFLYSKLTVDKSRWQQGNVFSCSVMHEALHNHYTQKSLSL
SPGK;
SEQ ID No: 41 (amino acid sequence of heavy chain constant region of human lgG4 type): 
ASTKGPSVFPLAPCSRSTSESTAALGCLVKDYFPEPVTVSWNSGALTSGVHTFPAVLQSSGLY
SLSSVVTVPSSSLGTKTYTCNVDHKPSNTKVDKRVESKYGPPCPPCPAPEFLGGPSVFLFPPKPK
DTLMISRTPEVTCVVVDVSQEDPEVQFNWYVDGVEVHNAKTKPREEQFNSTYRVVSVLTVLHQD
WLNGKEYKCKVSNKGLPSSIEKTISKAKGQPREPQVYTLPPSQEEMTKNQVSLTCLVKGFYPSDIA
VEWESNGQPENNYKTTPPVLDSDGSFFLYSRLTVDKSRWQEGNVFSCSVMHEALHNHYTQKSLS
LSLGK; and
SEQ ID No: 42 (amino acid sequence of light chain constant region of human Cₖ chain):


.

### Example 9 Preparation of chimeric antibody molecule

On basis of example 8, example 9 of the present invention further defined that a heavy chain constant region of a chimeric antibody molecule was a human IgG4 type, and the heavy chain constant region of the IgG4 type had an amino acid sequence shown in SEQ ID No: 41; and a light chain constant region of the chimeric antibody was the human Cₖ chain with an amino acid sequence shown in SEQ ID No: 42.

A specific preparation method was as follows:
The genes of the heavy chain variable region VH (SEQ ID No: 21) and light chain variable region VL (SEQ ID NO: 22) of the antibody molecule MA-1 screened from the phage antibody library of example 2 kept unchanged murine sequences, and were cloned into the pTSE vector (shown in Fig. 3) containing heavy chain and light chain constant region genes respectively, the heavy chain constant region was human IgG4 type (amino acid sequence shown in SEQ ID No: 41) and the light chain constant region was human Cₖ chain (amino acid sequence shown in SEQ ID No: 42). The vector was transiently transfected into HEK293E cells(purchased from the Institute of Basic Medicine of Chinese Academy of Medical Sciences, Cat. No:GNHu43) for expressing an antibody to obtain a chimeric antibody CA-1.

### Example 10 Humanization of murine antibody molecule MA-1

First, the sequence of the murine antibody molecule MA-1 in example 2 was compared with the human antibody germline database (v-base), human antibody light chain and heavy chain germlines with a relatively high homology were searched as candidate sequences, and then sequences of CDRs of the murine antibody molecule MA-1 were grafted onto a humanized candidate sequence for a homologous modeling. Then, the key framework amino acid residues that may play an important role in maintaining the CDR loop structure were calculated by three-dimensional structure simulation, so as to design the back mutation of the humanized antibody.

The designed humanized variable regions containing the back mutation of light chain and heavy chain were optimized and synthesized by GenScript Biotech Corporation (Nanjing), and then connected to a transient expression vector. The combination of the light chain and the heavy chain obtained by humanization was analyzed to obtain the following humanized antibody molecules: HA1, HA-2, HA-3, HA-4, and HA-5, wherein the sequences of the screened 5 monoclonal antibodies were as follows:

| Monoclonal antibody | Heavy chain variable region | Light chain variable region |
|---|---|---|
| HA-1 | SEQ ID No:33 | SEQ ID No:34 |
| HA-2 | SEQ ID No:33 | SEQ ID No:35 |
| HA-3 | SEQ ID No:36 | SEQ ID No:35 |
| HA-4 | SEQ ID No:36 | SEQ ID No:34 |
| HA-5 | SEQ ID No:37 | SEQ ID No:38 |

Specifically, SEQ ID No: 33 (amino acid sequence of heavy chain variable region of HA-1 and HA-2): 
QVQLVQSGAEVKKPGASVKVSCKASGYTFTDTYMHWVRQAPGQGLEWIGKIDPANGNTKYD
PKFQGRATITADTSISTAYMELSRLRSDDTAVYYCARDTTVGLDYWGQGTLVTVSS;
SEQ ID No: 34 (amino acid sequence of light chain variable region of HA-1 and HA-4): 
DIVITQSPAFLSVTPGEKVTITCRASSSVNYMYWYQQKPDASPKLWIYYTSKLASGVPSRFSG
SGSGTDYTFTISSLEAEDAATYYCQQWSSNPHTFGGGTKVEIK;
SEQ ID No: 35 (amino acid sequence of light chain variable region of HA-2 and HA-3): 
DIVITQSPAFLSVTPGEKVTITCRASSSVNYMYWYQQKPDQAPKLLIYYTSKLASGVPSRFSGS
GSGTDYTFTISSLEAEDAATYYCQQWSSNPHTFGGGTKVEIK;
SEQ ID No: 36 (amino acid sequence of heavy chain variable region of HA-3 and HA-4): 
QVQLVQSGAEVKKPGASVKVSCKASGYNITDTYMHWVRQAPGQGLEWIGKIDPANGNTKYD
PKFQGRATITADTSISTAYMELSRLRSDDTAVYYCARDTTVGLDYWGQGTLVTVSS;
SEQ ID No: 37 (amino acid sequence of heavy chain variable region of HA-5): 
QVQLVQSGAEVKKPGASVKVSCKASGYNIKDTYMHWVKQAPGQGLEWIGKIDPANGNTKYD
PKFQGKATITADTSISTAYMELSRLRSDDTAVYYCARDTTVGLDYWGQGTLVTVSS; and
SEQ ID No: 38 (amino acid sequence of light chain variable region of HA-5):

DIVITQSPAFLSVTPGEKVTITCRASSSVNYMYWYQQKPDASPKLWIYYTSKLASGVPARFSG
SGSGTSYTFTISSLEAEDAATYYCQQWSSNPHTFGGGTKVEIK.

### Example 11

On the basis of example 10, example 11 of the present invention further defined that the humanized antibody molecule further comprised a heavy chain constant region and a light chain constant region, the heavy chain constant region was one of human IgG1 type, IgG2 type, or IgG4 type, the heavy chain constant region of the IgG1 type had an amino acid sequence shown in SEQ ID No: 39, the heavy chain constant region of the IgG2 type had an amino acid sequence shown in SEQ ID No: 40, the heavy chain constant region of the IgG4 type had an amino acid sequence shown in SEQ ID No: 41, and the light chain constant region was a human Cₖ chain with an amino acid sequence shown in SEQ ID No: 42.

### Example 12 Preparation of humanized antibody molecule

On basis of example 11, example 12 of the present invention further defined that the heavy chain constant region of the humanized antibody molecule was human IgG4 type, and the heavy chain constant region of the IgG4 type had an amino acid sequence shown in SEQ ID No. 41; and a light chain constant region was the human Cₖ chain with an amino acid sequence shown in SEQ ID No. 42.

Encoding genes of heavy chain VH and light chain VL of the 5 humanized antibody molecules obtained by humanization in example 10 were cloned into a pTSE vector (see Fig. 3) containing heavy chain and light chain constant region genes respectively, a heavy chain constant region was the human IgG4 type (amino acid sequence shown in SEQ ID No: 41), and a light chain constant region was the Cₖ chain (amino acid sequence shown in SEQ ID No: 42)

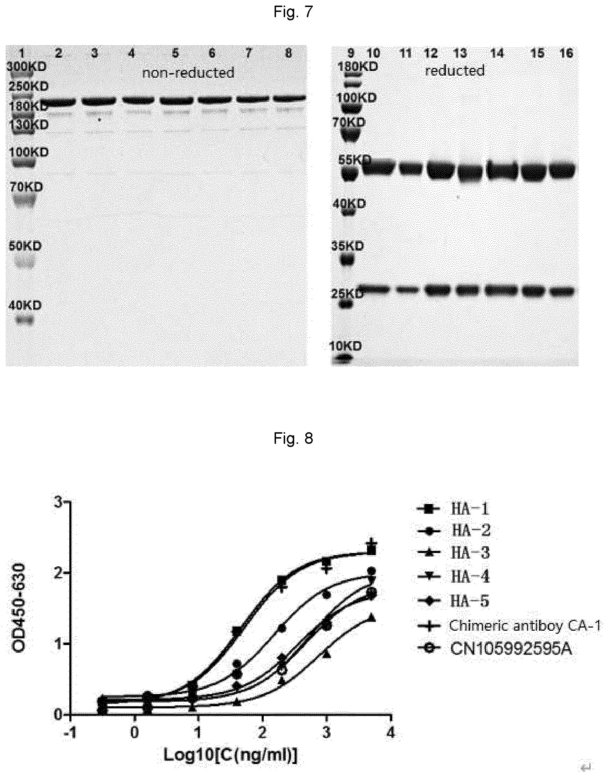

The chimeric antibody CA-1 and the humanized antibody were transiently transfected into HEK293E cells (purchased from the Institute of Basic Medicine of Chinese Academy of Medical Sciences, Cat. No. GNHu43) for expressing an antibody, monoclonal antibodies were obtained by a protein A affinity column purification using an AKTA instrument, a protein concentration was measured with a BCA kit (purchased from Beijing HuitianDongfang Technology Co., Ltd., Cat. No. BCA0020), and then a protein size was identified by SDS-PAGE. Results were shown in Fig. 7, non-reduced protein molecular weight Marker 1, HA-1, HA-2, HA-3, HA-4, HA-5, chimeric antibody CA-1 prepared in example 9, and anti-LAG-3 monoclonal antibody provided in core patent application CN105992595A, and reduced protein molecular weight Marker 2, HA-1, HA-2, HA-3, HA-4, HA-5, chimeric antibody CA-1, and anti-LAG-3 monoclonal antibody provided in core patent application CN105992595A were sequentially present from left to right, and the molecular weight of each band was consistent with a theoretical value.

### Example 13

On the basis of the above example, example 13 of the present invention further defined that the humanized antibody molecule was a full-length antibody or an antibody fragment, and comprised one or a combination of more of Fab, F(ab)2, Fv or ScFv.

### Example 14

On the basis of the above example, example 14 of the present invention further defined the following solution.

A polypeptide or a protein comprising the anti-LAG-3 monoclonal antibody or the antigen binding fragment thereof defined by any one of the above examples was further defined.

A polynucleotide sequence or combination encoding an amino acid sequence of the anti-LAG-3 monoclonal antibody or the antigen binding fragment thereof defined by any one of the above examples was further defined.

A recombinant DNA expression vector comprising the polynucleotide sequence or combination was further defined.

A host cell transfected with the defined recombinant DNA expression vector was further defined, wherein the cell comprised a prokaryotic cell, a yeast cell, an insect cell, or a mammalian cell; and
preferably, the host cell was the mammalian cell, and the mammalian cell was an HEK293E cell, a CHO cell, or an NS0 cell.

A drug or pharmaceutical composition comprising the anti-LAG-3 monoclonal antibody or the antigen-binding defined by any one of the above examples was further defined.

The present invention further provided use of the anti-LAG-3 monoclonal antibody or the antigen binding fragment thereof in the preparation of a drug for treating a cancer or an immunological disease; and
preferably, the cancer included but was not limited to leukemia, lung cancer, stomach cancer, esophageal cancer, ovarian cancer, head and neck cancer, melanoma, kidney cancer, breast cancer, colorectal cancer, liver cancer, pancreatic cancer or bladder cancer; and the immunological disease included but was not limited to psoriasis, Crohn's disease, rheumatoid arthritis, primary biliary cirrhosis, systemic lupus erythematosus, multiple sclerosis, ulcerative colitis, and autoimmune hepatitis.

### Example 15 Binding experiment of humanized antibody molecule to LAG-3

100ng/well/100µl of LAG-3 was coated with a carbonate buffer solution at a pH of 9.6 overnight at 4°C. The LAG-3 was washed five times with 300µl/well of PBST, then 1% of BSA-PBST was added, the LAG-3 was blocked at 37°C for 2h, the humanized antibodies of HA-1, HA-2, HA-3, HA-4, and HA-5, the chimeric antibody CA-1 prepared in example 9, and the anti-LAG-3 antibody in patent application CN105992595A with different dilution concentrations were added, wherein the 7 antibodies all had an initial highest concentration of 5µg/ml and respectively diluted by 5 times, and each antibody had 8 gradients, and the mixture was incubated at 37°C for 1 h. The obtained product was washed five times with 300 µl/well of PBST, then Anti-Human Fc-HRP diluted with 1% BSA-PBST at 1:10,000 was added, and the mixture was incubated at 37°C for 1 h. Color development was performed with 100µl/well of a TMB color developing kit at room temperature for 8 minutes, and terminated with 2M of H₂SO₄. Reading was performed at 450 nm/630 nm, a corresponding EC50 value was calculated, and specific data was as follows:

| Clone | HA-1 | HA-2 | HA-3 | HA-4 | HA-5 | Chimeric antibody CA-1 | CN105992595A |
|---|---|---|---|---|---|---|---|
| EC50 (ng/ml) | 46.25 | 150.7 | 102.5 | 538.8 | 350.3 | 52.16 | 747.2 |

Based on the data and experiment result shown in Fig. 8, the 5 different humanized antibody molecules may all bind to LAG-3. The EC50 values of the 5 different monoclonal antibodies provided by the present invention were all significantly lower than that of the anti-LAG-3 antibody in patent application CN105992595A, which indicated that the monoclonal antibodies provided by the present invention had a strong binding ability to the LAG-3 and a high affinity. Besides, it may also be seen from Fig. 8 and the data that the EC50 value of the HA-1 among the 5 different monoclonal antibodies was the lowest, which indicated that the monoclonal antibody has a best binding ability to the LAG-3 and a highest affinity. Meanwhile, the EC50 value of the HA-1 was similar to that of the chimeric antibody CA-1, which indicated that the humanized HA-1 retained the high affinity of the murine parent antibody MA-1 and the LAG-3, and the affinity was not reduced.

### Example 16 Activity of anti-LAG-3 humanized antibody molecule tested by mixed lymphocyte reaction (MLR)

Fresh peripheral blood mononuclear cells (PBMCs) were separated by a density gradient centrifugation and CD14⁺ T cells were sorted by magnetic beads; and the CD14⁺ T cells were cultured by a culture medium of 20ng/mL of GM-CSF and 10ng/mL of IL-4, the culture medium was changed every two days, and the cells were induced into dendritic cells (DCs) in 7-10 days. Two days before the DCs were used, 25ng/mL of TNF-α was added to induce the DCs into mature DCs, and the mature DCs were collected and prepared into a cell suspension with a cell density of 1×10⁵ cells/mL.The CD14⁺ T cells were sorted from the PBMCs with magnetic beads, counted, and prepared into a cell suspension with a cell density of 1×10⁶ cells/mL. 100µl of each of the CD14⁺ T cells and the DCs were taken and added into a 96-well plate according to a ratio of 10:1.

The 5 anti-LAG-3 humanized antibody molecules prepared in example 12, the chimeric antibody CA-1 prepared in example 9, and the anti-LAG-3 antibody provided in patent application CN105992595A were used as positive controls respectively subjected to a 4-fold gradient dilution, 8 gradients were set for each antibody, 50µl of each antibody was taken respectively and added into a 96-well plate, after 5 days, CD14⁺ T cell proliferation was tested by CCK8, reading was performed at 450nm/630nm, a corresponding EC50 value was calculated, and specific data was as follows:

| Clone | HA-1 | HA-2 | HA-3 | HA-4 | HA-5 | Chimeric antibody CA-1 | DN105992595A |
|---|---|---|---|---|---|---|---|
| EC50(ng/ml) | 6.875 | 27.09 | 15.01 | 33.8 | 31.84 | 7.226 | 39.59 |

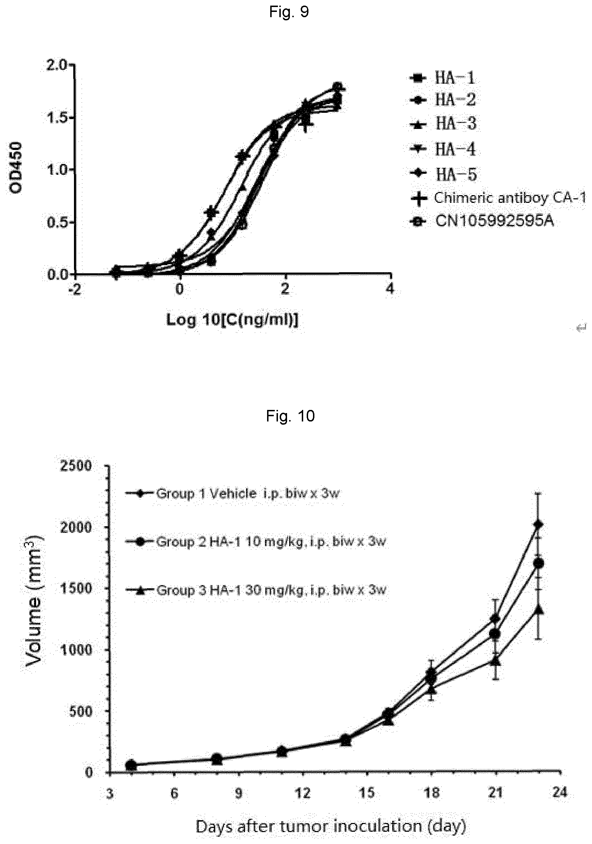

Based on the data and Fig. 9, the EC50 values of the 5 different anti-LAG-3 antibodies screened by the present invention were all significantly lower than that of the anti-LAG-3 antibody provided in patent application CN105992595A, which indicated that the anti-LAG-3 antibodies provided by the present invention all had higher activity. Meanwhile the anti-LAG-3 whole antibody HA-1 had the lowest EC50 value among the 5 screened monoclonal antibodies, which indicated the antibody had a highest activity. In addition, it may be further seen from Fig. 9 that the EC50 value of the chimeric antibody CA-1 was closer to that of the anti-LAG-3 antibody HA-1, which indicated that the humanized antibody HA-1 retained the biological activity of the murine parent antibody MA-1, and the biological activity thereof was not reduced.

### Example 17 Inhibition test of anti-LAG-3 monoclonal antibody HA-1 on MC38 colorectal cancer in mouse

1. Experimental animal:
   species strain: Mus Musculus, NCG, mouse;
   week age: 6-8 weeks; and
   experimental animal provider: GemPharmatech Co., Ltd.
2. Cell culture: MC38 tumor cells (purchased from Biovector NTCC Inc., Cat. No. NTCC-MC38). The tumor cells were cultured with a DMEM containing inactivated 10% fetal bovine serum (ExCell Bio., Cat. No. FND500), 100 U/ml of penicillin, 100µg/ml of streptomycin, and 2mM of glutamine (purchased from ThermoFisher Scientific (China) Co., Ltd. (Gibco), Cat. No. 10566-016) in a 5% CO₂ incubator at 37°C, and passaged in vials every other 3-4 days after the cell grew full, and the tumor cells in a logarithmic growth phase were used for tumor inoculation *in vivo.*
3. Inoculation of tumor cells and grouping: the PBS-resuspended MC38 tumor cells with a concentration of 1.0×10⁷/mL were inoculated subcutaneously in the right rib of the experimental animal at 100µL per animal. When the tumor grew to about 61mm³, the mice were administered in 3 groups with 8 mice in each group. The groups were a vehicle control group, HA-1 (10 mg/kg, i.p., biw×3w), and HA-1 (30 mg/kg, i.p., biw×3 w).
4. Detection indicators: the tumor volume was measured twice a week using a vernier caliper, the long diameter and the short diameter of the tumor were measured, and the volume calculation formula was: volume = 0.5×long diameter r×short diameter², and a relationship between the change in the tumor volume and the administration time was recorded and the result was shown in Fig. 10.

Based on data in Fig. 10, the anti-LAG-3 monoclonal antibody HA-1 may inhibit tumor growth, exhibiting a dose-dependent response.

### Example 18 Evaluation of thermostability of anti-LAG-3 monoclonal antibody HA-1 protein molecule

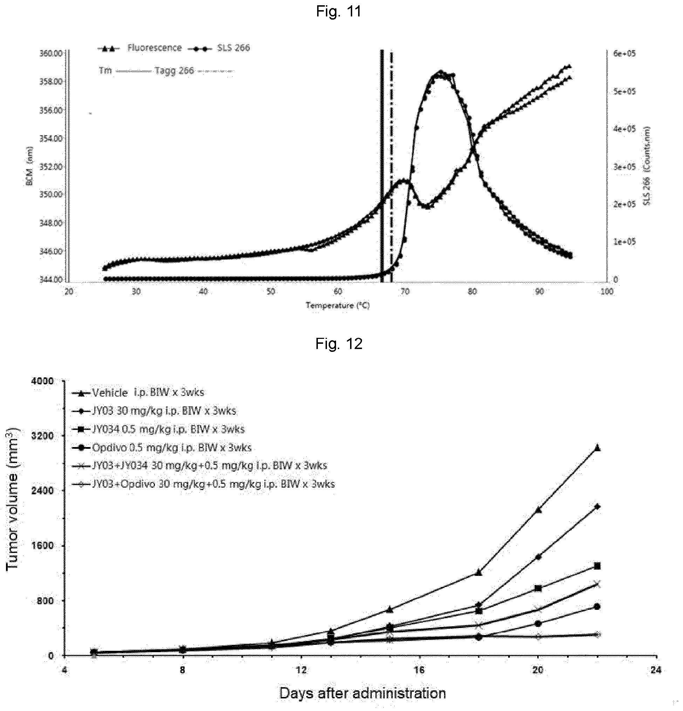

The anti-LAG-3 monoclonal antibody HA-1 protein molecule was ultrafiltered, transferred in a PBS buffer system, and centrifuged at 12,000 rpm and 4°C for 5min, and the thermostability of the anti-LAG-3 monoclonal antibody HA-1 protein molecule was evaluated by a multifunctional protein thermostability analysis system (purchased from Uncariained Labs). By monitoring the change of the protein endogenous fluorescence with a temperature (from 25°C, heating to 95°C at a heating rate of 0.3 °C/min),the change of a protein conformation was detected, a protein melting temperature Tm was determined and the protein conformation stability was evaluated. When a sample was aggregated, a scattered light wave was interfered and a signal of the scattered light was increased. A colloidal stability of a protein (characterized by Tagg) was determined by a static light scattering. The result was shown in the following table and Fig. 11.

| Sample | Temperature (°C) | Average temperature (°C) | Tagg 266 (°C) | Average Tagg 266 (°C) |
|---|---|---|---|---|
| 2 mg/ml of anti-LAG-3 monoclonal antibody HA-1 protein molecule | 66.6 | 66.6 | 67.96 | 68.0 |
| | 66.6 | | 68.02 | |

As shown in the above table and Fig. 11, the anti-LAG-3 monoclonal antibody HA-1 protein molecule had a temperature of 66.6°C and an average Tagg of 68.0°C and showed a superior conformational stability and colloidal stability.

### Example 19

On the basis of the above example, example 19 of the present invention further defined the following solution:
The present invention further provided use of the anti-LAG-3 monoclonal antibody or the antigen binding fragment thereof in combination with an anti-PD-1 monoclonal antibody in the preparation of a drug for treating a cancer or an immunological disease.

Further, the anti-PD-1 monoclonal antibody was selected from DFPD1-9, DFPD1-10, DFPD1-11, DFPD1-12, DFPD1-13, nivolumab, pembrolizumab, toripalimab, sintilimab, tislelizumab, camrelizumab, penpulimab, or zimberelimab.

The nivolumab, pembrolizumab, toripalimab, sintilimab, tislelizumab, camrelizumab, penpulimab, or zimberelimab were all marketed products.

The DFPD1-9, DFPD1-10, DFPD1-11, DFPD1-12, and DFPD1-13 were anti-PD-1 monoclonal antibodies provided by a patent with the application number of CN201510312910.8, the DFPD1-9 comprised a light chain variable region shown in SEQ ID No: 44 and a heavy chain variable region shown in SEQ ID No: 43; the DFPD1-10 comprised a light chain variable region shown in SEQ ID No: 45 and a heavy chain variable region shown in SEQ ID No: 43; the DFPD1-11 comprised a light chain variable region shown in SEQ ID No: 44 and a heavy chain variable region shown in SEQ ID No: 46; the DFPD1-12 comprised a light chain variable region shown in SEQ ID No: 44 and a heavy chain variable region shown in SEQ ID No: 47; and the DFPD1-13 comprised a light chain variable region shown in SEQ ID No: 45 and a heavy chain variable region shown in SEQ ID No: 46. Specific sequences were as follows:

A sequence of SEQ ID No. 43 was as follows:

QVQLVESGGGVVQPGRSLRLDCKASGITFSNYGMHWVRQAPGKGLEWVAVIWYDSSRKYY
ADSVKGRFTISRDNSKNTLFLQMNSLRAEDTAVYYCATNNDYWGQGTLVTVSS;

A sequence of SEQ ID No. 44 was as follows:

DIQMTQSPSSLSASVGDRVTITCRASQSIHNYLDWYQQKPGKAPKLLIYNASTRATGVPSRFS
GSGSGTDFTLTISSLQPEDFATYYCQQELHLPLTFGQGTKVEIK;

A sequence of SEQ ID No. 45 was as follows:

DIQMTQSPSSLSASVGDRVTITCRASQSVSNYLDWYQQKPGKAPKLLIYDASTRATGVPSRF
SGSGSGTDFTLTISSLQPEDFATYYCQQNMQLPLTFGQGTKVEIK;

A sequence of SEQ ID No. 46 was as follows:

QVQLVESGGGVVQPGRSLRLDCKASGITFSNNGMHWVRQAPGKGLEWVAVIWYDSSRKYY
ADSVKGRFTISRDNSKNTLFLQMNSLRAEDTAVYYCATNNDYWGQGTLVTVSS; and

A sequence of SEQ ID No. 47 was as follows:

QVQLVESGGGVVQPGRSLRLDCKASGITFSNYGMHWVRQAPGKGLEWVAVIWYDGSKKYY
ADSVKGRFTISRDNSKNTLFLQMNSLRAEDTAVYYCATNNDYWGQGTLVTVSS.

In addition, the anti-PD-1 monoclonal antibody further comprised a heavy chain constant region and a light chain constant region. The sequences of the heavy chain constant region and the light chain constant region were the same as those of the heavy chain constant region and the light chain constant region of the anti-PD-1 monoclonal antibody provided in the patent with the application number of CN201510312910.8.

Further, the cancer was selected from leukemia, lung cancer, stomach cancer, esophageal cancer, ovarian cancer, head and neck cancer, melanoma, kidney cancer, breast cancer, colorectal cancer, liver cancer, pancreatic cancer or bladder cancer; and the immunological disease comprised psoriasis, Crohn's disease, rheumatoid arthritis, primary biliary cirrhosis, systemic lupus erythematosus, multiple sclerosis, ulcerative colitis, and autoimmune hepatitis.

### Example 20

On the basis of example 19, example 20 of the present invention further defined that the anti-PD-1 monoclonal antibody was DFPD1-10, and the DFPD1-10 comprised a light chain variable region shown in SEQ ID No: 45 and a heavy chain variable region shown in SEQ ID No: 43.

### Example 21

On the basis of example 19, example 21 of the present invention further defined that the anti-PD-1 monoclonal antibody was nivolumab.

### Example 22 In-vivo pharmaceutical effect experiment of anti-LAG-3 monoclonal antibody in combination with anti-PD-1 monoclonal antibody for MC38 colorectal cancer model

### 1. Test drug

(1) The anti-LAG-3 monoclonal antibody HA-1 provided in example 10 with the number of JY03;
   provider: Beijing Dongfang Biotech Co., Ltd; and
   quantity: 11mL per one, 4.9mg/mL, and a total of 7;
(2) The anti-PD-1 monoclonal antibody provided in example 20 was DFPD1-10 with the number of JY034 in the experiment;
   provider: Beijing Dongfang Biotech Co., Ltd; and
   quantity: 10ml,10mg/ml, 3 bottles, and a total of 300mg; and
(3) Opdivo (nivolumab)
   manufacturer: Bristol Myers Squibb; and
   quantity: 100mg/10ml,1

### 2. Experimental animal

Species strain: MusMusculus, B6/JGpt-PdcdlemICin (hPDCD1) Lag3em 1 Cin (hLAG3)/Gpt
Sex: female
Week age: 6-8 weeks
Weight: 18-22g
Quantity: 75
Experimental animal provider: GemPharmatech Co. Ltd.
Production license number: SCXK (Su) 2018-0008

### 3. Experimental method

(1) Cell culture
   MC38 tumor cell was cultured with a DMEM containing inactivated 10% fetal bovine serum, 100U/mL of penicillin, 100µg/mL of streptomycin, and 2mM of glutamine in a 5% CO₂ incubator at 37°C, and passaged in vials every other 3-4 days after the cell grew full, and the tumor cell in a logarithmic growth phase was used for tumor inoculation *in vivo.*
(2) Tumor cell inoculation and grouping
   The PBS-resuspended MC38 tumor cells with a concentration of 1.0×10⁷/mL was inoculated subcutaneously in the right rib of the experimental animal at 1×10⁶/100µL per animal. When the tumor grew to about 50mm³, the mice were administered (the same day was marked PG-D0) in 8 groups with 10 mice in each group.
(3) Measurement of mouse weight and experiment indicators
   A tumor volume was measured 2-3 times a week using a vernier caliper, a mouse weight was weighed using an electronic scale, long diameter and short diameter of the tumor were measured, and a volume calculation formula was: volume = 0.5×long diameter ×short diameter². The T/C value was calculated according to the tumor volume, wherein T is an average value of a relative tumor volume (RTV) of each test substance-treated group, C is an average value of a RTV of a control group, and the RTV is a ratio of the tumor volume after administration to that before administration. Tumor growth inhibition rate (TGITV) (%) = (1-T/C) ×100%.

At an end of the experiment, the animals were euthanized, the tumors were stripped, weighed, placed in order, and photographed. The tumor weight inhibition rate (TGITW) (%) was calculated with a formula (1-T/C)×100%, wherein T/C = TW average value of treatment groups/TW average value of control group.

In principle, an evaluation criterion was: T/C (%) > 40% was ineffective; and T/C (%) was less than or equal to 40%, and P< 0.05 was effective after a statistical treatment.

### 4. Administration regimen

| Groups | Number of animals | Treatment | Dose (mg/kg)* | Administrati on route | Administration frequency |
|---|---|---|---|---|---|
| 1 | 10 | Vehicle | - | i.p. | biw × 3 wks |
| 2 | 10 | JY03 | 30 | i.p. | biw × 3 wks |
| 3 | 10 | JY034 | 0.5 | i.p. | biw × 3 wks^{#} |
| 4 | 10 | Opdivo | 0.5 | i.p. | biw × 3 wks^{#} |
| 5 | 10 | JY03 | 30 | i.p. | biw × 3 wks |
| | | JY034 | 0.5 | | biw × 3 wks^{#} |
| 6 | 10 | JY03 | 30 | i.p. | biw × 3 wks |
| | | Opdivo | 0.5 | | biw × 3 wks^{#} |

| | | | | | |
|---|---|---|---|---|---|
| Note: *, an administration volume of each group was 10 µL/g according to an animal weight and the administration amount may be adjusted when the weight was reduced by 15-20%; i.p.: intraperitoneal injection; biw×3 wks: twice weekly for three weeks, and a total of 6 times; and ^{#}: a drug administration was started at a second administration time of JY03 and the actual administration time was a total of 5 times in three weeks. | | | | | |

### 5. Statistical analysis

A statistical analysis among groups was performed on tumor volume and tumor weight using an IBM SPSS Statistics 22.0 statistical software and a one-way ANOVA test. A significant difference was considered when P< 0.05.

### 6. Data results.

(1) Results of tumor growth inhibition:

| Groups | Number of animals | Body weight (g)³ | | Tumor volume (mm³)² | | T/C (%) | Tumor growth inhibition rate (%) | *p^{b}* | *_{P}^{c}* | *P^{d}* | *P^{e}* | *P^{f}* |
|---|---|---|---|---|---|---|---|---|---|---|---|---|
| | | Before administrati on | After administrati on | Before administrati on | After administrati on | | | | | | | |
| | | (PG-D0) | (PG-D17) | (PG-D0) | (PG-D17) | | | | | | | |
| Vehicle | 10 | 21.1±0.4 | 25.4±0.8 | 50±3 | 3032±577 | - | - | - | <0.001 | 0.149 | 0001 | <0,00 1 |
| JY03 | 10 | 21.8±0.5 | 25.2±0.5 | 50±3 | 2173±339 | 75% | 25% | 0.149 | 0.016 | - | 0.059 | 0.002 |
| JY034 | 10 | 21.3±0.3 | 24.6±0.9 | 50±3 | 1308±487 | 42% | 58% | 0.005 | 0.316 | 0.146 | 0.653 | 0.095 |
| Opdivo | 10 | 21.2±0.2 | 22.6±0.7 | 50±3 | 713±478 | 28% | 72% | <0.001 | | 0.016 | 0.579 | 0.497 |
| JY03+JY034 | 10 | 21.1±0.3 | 23.8+0.8 | 50±3 | 1041±331 | 38% | 62% | 0.001 | 0.579 | 0.059 | - | 0.219 |
| JY03+Opdivo | 10 | 21.0±0.5 | 22.4±0.5 | 49±3 | 311±129 | 10% | 90% | <0.001 | 0.497 | 0.002 | 0.219 | |

| | | | | | | | | | | | | |
|---|---|---|---|---|---|---|---|---|---|---|---|---|
| Note: A one-way ANOVA was performed on each group, a least significant difference (LSD) was used for a post hoc test; ^{a}, mean±standard errors; ^{b}, compared with the vehicle group; ^{c}, compared with the Opdivo group; ^{d}, compared with the JY03 group; ^{e}, compared with the JY03+JY034 group; and ^{f}, compared with the JY03+Opdivo group. There were no statistical differences among the JY034 treatment groups (all P> 0.05). 3, 5, 1, and 4 mice had completely regressed tumors in the JY034 group, the Opdivo group, the JY03+JY034 group, and the JY03+Opdivo group, respectively. | | | | | | | | | | | | |

(2) Tumor weight

| Groups | Number of animals | Tumor weight (g)^{a} | Tumor weight Inhibition rate (%) | *p^{b}* | *P*^{c} | *P^{d}* | *P^{e}* | *P^{f}* |
|---|---|---|---|---|---|---|---|---|
| Vehicle | 10 | 2.980±0.596 | - | - | <0.001 | 0.255 | 0.003 | <0.001 |
| JY03 | 10 | 2.254±0.379 | 24% | 0.255 | 0.027 | - | 0.057 | 0.002 |
| JY034 | 10 | 1.318±0.500 | 56% | 0.010 | 0.443 | 0.143 | 0.655 | 0.097 |
| Opdivo | 10 | 0.830±0.541 | 72% | 0.001 | - | 0.027 | 0.748 | 0.367 |
| JY03+JY034 | 10 | 1.034±0.349 | 65% | 0.003 | 0.748 | 0.057 | - | 0.223 |
| JY03+Opdivo | 10 | 0.256±0.113 | 91% | <0.001 | 0.367 | 0.002 | 0.223 | - |

| | | | | | | | | |
|---|---|---|---|---|---|---|---|---|
| Note: A one-way ANOVA was performed on each group, a least significant difference (LSD) was used for a post hoc test; ^{a}, mean±standard errors; ^{b}, compared with the vehicle group; ^{c}, compared with the Opdivo group; ^{d}, compared with the JY03 group; ^{e}, compared with the JY03+JY034 group; and ^{f}, compared with the JY03+Opdivo group. | | | | | | | | |

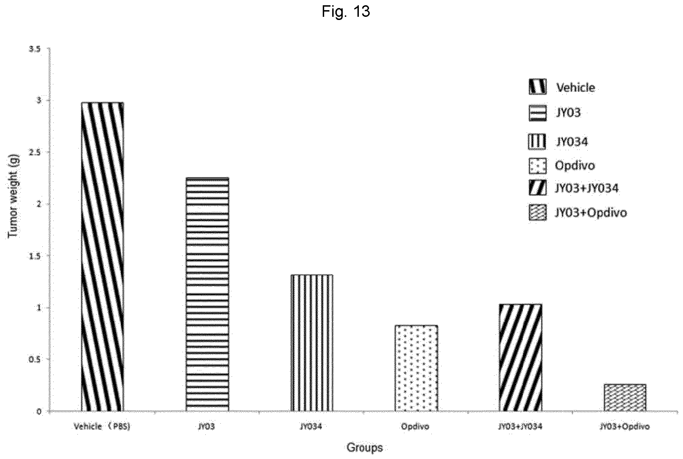

In conclusion, based on the data and Figs. 12 and 13, in the test drug JY03 group, JY034 group, Opdivo group, JY03+JY034 group, and JY03+Opdivo group, a definite anti-tumor effect was generated on a PD-1 and LAG3 double-target humanized colorectal cancer MC38 model, wherein a tumor inhibition effect of the JY03+JY034 group was obviously stronger than that of the single drug JY03 group and the single drug JY034 group, and the tumor inhibition effect of the JY03+Opdivo group was obviously stronger than that of the single drug JY03 group and the single drug Opdivo group. Therefore, the anti-LAG-3 monoclonal antibody may improve the tumor inhibition effect of the anti-PD-1 monoclonal antibody. In addition, the animals in each group had a good tolerance and no obvious adverse reaction was shown.

The present invention is not limited to the above optimal embodiments, and anyone may derive various other forms of products under the motivation of the present invention. However, regardless of any changes in their shapes or structures, any technical solution that is the same or similar to the present application falls within the scope of protection of the present invention.

## Claims

1. An anti-LAG-3 monoclonal antibody or an antigen binding fragment thereof, comprising a heavy chain variable region and a light chain variable region, wherein the heavy chain variable region comprises 3 heavy chain complementarity determining regions, respectively represented by HCDR1, HCDR2, and HCDR3, and the light chain variable region comprises 3 light chain complementarity determining regions, respectively represented by LCDR1, LCDR2, and LCDR3, and the monoclonal antibody or the antigen binding fragment thereof is selected from any one of the following:
A-1: the heavy chain complementarity determining region HCDR1 comprises an amino acid sequence shown in SEQ ID No: 1, the heavy chain complementarity determining region HCDR2 comprises an amino acid sequence shown in SEQ ID No: 2, the heavy chain complementarity determining region HCDR3 comprises an amino acid sequence shown in SEQ ID No: 3, the light chain complementarity determining region LCDR1 comprises an amino acid sequence shown in SEQ ID No: 4, the light chain complementarity determining region LCDR2 comprises an amino acid sequence shown in SEQ ID No: 5, and the light chain complementarity determining region LCDR3 comprises an amino acid sequence shown in SEQ ID No: 6;
A-2: the heavy chain complementarity determining region HCDR1 comprises an amino acid sequence shown in SEQ ID No: 1, the heavy chain complementarity determining region HCDR2 comprises an amino acid sequence shown in SEQ ID No: 2, the heavy chain complementarity determining region HCDR3 comprises an amino acid sequence shown in SEQ ID No: 3, the light chain complementarity determining region LCDR1 comprises an amino acid sequence shown in SEQ ID No: 4, the light chain complementarity determining region LCDR2 comprises an amino acid sequence shown in SEQ ID No: 7, and the light chain complementarity determining region LCDR3 comprises an amino acid sequence shown in SEQ ID No: 8;
A-3: the heavy chain complementarity determining region HCDR1 comprises an amino acid sequence shown in SEQ ID No: 9, the heavy chain complementarity determining region HCDR2 comprises an amino acid sequence shown in SEQ ID No: 10, the heavy chain complementarity determining region HCDR3 comprises an amino acid sequence shown in SEQ ID No: 11, the light chain complementarity determining region LCDR1 comprises an amino acid sequence shown in SEQ ID No: 12, the light chain complementarity determining region LCDR2 comprises an amino acid sequence shown in SEQ ID No: 13, and the light chain complementarity determining region LCDR3 comprises an amino acid sequence shown in SEQ ID No: 14; and
A-4: the heavy chain complementarity determining region HCDR1 comprises an amino acid sequence shown in SEQ ID No: 15, the heavy chain complementarity determining region HCDR2 comprises an amino acid sequence shown in SEQ ID No: 16, the heavy chain complementarity determining region HCDR3 comprises an amino acid sequence shown in SEQ ID No: 17, the light chain complementarity determining region LCDR1 comprises an amino acid sequence shown in SEQ ID No: 18, the light chain complementarity determining region LCDR2 comprises an amino acid sequence shown in SEQ ID No: 19, and the light chain complementarity determining region LCDR3 comprises an amino acid sequence shown in SEQ ID No: 20.

2. The anti-LAG-3 monoclonal antibody or the antigen binding fragment thereof according to claim 1, wherein the monoclonal antibody or the antigen binding fragment thereof is a murine antibody molecule, and the murine antibody molecule is selected from any one of the following:
MA-1: the heavy chain variable region comprises an amino acid sequence shown in SEQ ID No: 21, and the light chain variable region comprises an amino acid sequence shown in SEQ ID No: 22;
MA-2: the heavy chain variable region comprises an amino acid sequence shown in SEQ ID No: 21, and the light chain variable region comprises an amino acid sequence shown in SEQ ID No: 23;
MA-3: the heavy chain variable region comprises an amino acid sequence shown in SEQ ID No: 24, and the light chain variable region comprises an amino acid sequence shown in SEQ ID No: 25; and
MA-4: the heavy chain variable region comprises an amino acid sequence shown in SEQ ID No: 26, and the light chain variable region comprises an amino acid sequence shown in SEQ ID No: 27; and
preferably, the murine antibody molecule is the MA-1.

3. The anti-LAG-3 monoclonal antibody or the antigen binding fragment thereof according to claim 2, wherein the murine antibody molecule further comprises a heavy chain constant region and a light chain constant region, the heavy chain constant region is one of murine IgG1 type, !gG2a type, IgG2b type, and IgG3 type, the heavy chain constant region of the IgG1 type has an amino acid sequence shown in SEQ ID No: 29, the heavy chain constant region of the IgG2a type has an amino acid sequence shown in SEQ ID No: 30, the heavy chain constant region of the IgG2b type has an amino acid sequence shown in SEQ ID No: 31, and the heavy chain constant region of the IgG3 type has an amino acid sequence shown in SEQ ID No: 32; and the light chain constant region is a murine Cₖ chain with an amino acid sequence shown in SEQ ID No: 28; and
preferably, the heavy chain constant region is the murine IgG1 type.

4. The anti-LAG-3 monoclonal antibody or the antigen binding fragment thereof according to claim 2, wherein the monoclonal antibody or the antigen binding fragment thereof is a chimeric antibody molecule, the chimeric antibody molecule comprises a heavy chain variable region of the murine antibody molecule, a light chain variable region of the murine antibody molecule, and a humanized antibody constant region; the humanized antibody constant region comprises a humanized antibody heavy chain constant region and a humanized antibody light chain constant region, the humanized antibody heavy chain constant region is one of human IgG1 type, IgG2 type, or IgG4 type, the humanized antibody heavy chain constant region of the IgG1 type has an amino acid sequence shown in SEQ ID No: 39, the humanized antibody heavy chain constant of the IgG2 typeregion has an amino acid sequence shown in SEQ ID No: 40, and the humanized antibody heavy chain constant region of the IgG4 type has an amino acid sequence shown in SEQ ID No: 41; and the humanized antibody light chain constant region is a human Cₖ chain having an amino acid sequence shown in SEQ ID No: 42; and
preferably, the humanized antibody heavy chain constant region is the human IgG4 type.

5. The anti-LAG-3 monoclonal antibody or the antigen binding fragment thereof according to claim 1, wherein the monoclonal antibody or the antigen binding fragment thereof is a humanized antibody molecule, and the humanized antibody molecule is selected from any one of the following:
HA-1: the heavy chain variable region comprises an amino acid sequence shown in SEQ ID No: 33, and the light chain variable region comprises an amino acid sequence shown in SEQ ID No: 34;
HA-2: the heavy chain variable region comprises an amino acid sequence shown in SEQ ID No: 33, and the light chain variable region comprises an amino acid sequence shown in SEQ ID No: 35;
HA-3: the heavy chain variable region comprises an amino acid sequence shown in SEQ ID No: 36, and the light chain variable region comprises an amino acid sequence shown in SEQ ID No: 35;
HA-4: the heavy chain variable region comprises an amino acid sequence shown in SEQ ID No: 36, and the light chain variable region comprises an amino acid sequence shown in SEQ ID No: 34; and
HA-5: the heavy chain variable region comprises an amino acid sequence shown in SEQ ID No: 37, and the light chain variable region comprises an amino acid sequence shown in SEQ ID No: 38; and
preferably, the humanized antibody molecule is the HA-1.

6. The anti-LAG-3 monoclonal antibody or the antigen binding fragment thereof according to claim 5, wherein the humanized antibody molecule further comprises a heavy chain constant region and a light chain constant region, the heavy chain constant region is one of human IgG1 type, IgG2 type, or IgG4 type, the heavy chain constant region of the IgG1 type has an amino acid sequence shown in SEQ ID No: 39, the heavy chain constant region of the IgG2 type has an amino acid sequence shown in SEQ ID No: 40, the heavy chain constant region of the IgG4 type has an amino acid sequence shown in SEQ ID No: 41, and the light chain constant region is a human Cₖ chain with an amino acid sequence shown in SEQ ID No: 42; and
preferably, the heavy chain constant region is the human IgG4 type.

7. The anti-LAG-3 monoclonal antibody or the antigen binding fragment thereof according to claim 5, wherein the humanized antibody molecule is a full-length antibody or an antibody fragment,the humanized antibody molecule comprises one or a combination of more of Fab, F(ab)2, Fv or ScFv.

8. A polypeptide or a protein, comprising the anti-LAG-3 monoclonal antibody or the antigen binding fragment thereof according to any one of claims 1-7.

9. A polynucleotide sequence or combination, encoding an amino acid sequence of the anti-LAG-3 monoclonal antibody or the antigen binding fragment thereof according to any one of claims 1-7.

10. A recombinant DNA expression vector, comprising the polynucleotide sequence or combination according to claim 9.

11. A host cell transfected with the recombinant DNA expression vector according to claim 10, wherein the host cell comprises a prokaryotic cell, a yeast cell, an insect cell, or a mammalian cell; and
preferably, the host cell is the mammalian cell, and the mammalian cell is an HEK293E cell, a CHO cell, or an NS0 cell.

12. A drug or a pharmaceutical composition, comprising the anti-LAG-3 monoclonal antibody or the antigen binding fragment thereof according to any one of claims 1-7.

13. Use of the anti-LAG-3 monoclonal antibody or the antigen binding fragment thereof according to any one of claims 1-7 in the preparation of a drug for treating a cancer or an immunological disease;
preferably, the cancer comprises leukemia, lung cancer, stomach cancer, esophageal cancer, ovarian cancer, head and neck cancer, melanoma, kidney cancer, breast cancer, colorectal cancer, liver cancer, pancreatic cancer or bladder cancer; and
the immunological disease comprises psoriasis, Crohn's disease, rheumatoid arthritis, primary biliary cirrhosis, systemic lupus erythematosus, multiple sclerosis, ulcerative colitis, and autoimmune hepatitis.

14. Use of the anti-LAG-3 monoclonal antibody or the antigen binding fragment thereof according to any one of claims 1-7 in combination with an anti-PD-1 monoclonal antibody in the preparation of a drug for treating a cancer or an immunological disease.

15. The use according to claim 14, wherein the anti-PD-1 monoclonal antibody is selected from the group consisting of DFPD1-9, DFPD1-10, DFPD1-11, DFPD1-12, DFPD1-13, nivolumab, pembrolizumab, toripalimab, sintilimab, tislelizumab, camrelizumab, penpulimab, or zimberelimab.

16. The use according to claim 15, wherein the anti-PD-1 monoclonal antibody is DFPD1-10, and the DFPD1-10 comprises a light chain variable region shown in SEQ ID No: 45 and a heavy chain variable region shown in SEQ ID No: 43.

17. The use according to claim 15, wherein the anti-PD-1 monoclonal antibody is nivolumab.

18. The use according to claim 14, wherein the cancer is selected from leukemia, lung cancer, stomach cancer, esophageal cancer, ovarian cancer, head and neck cancer, melanoma, kidney cancer, breast cancer, colorectal cancer, liver cancer, pancreatic cancer or bladder cancer; and the immunological disease comprises psoriasis, Crohn's disease, rheumatoid arthritis, primary biliary cirrhosis, systemic lupus erythematosus, multiple sclerosis, ulcerative colitis, and autoimmune hepatitis.
